Europäisches Patentamt

European Patent Office

Office européen des brevets

(19)

(11) **EP 1 047 987 B1**

(12) **EUROPÄISCHE PATENTSCHRIFT**

(45) Veröffentlichungstag und Bekanntmachung des Hinweises auf die Patenterteilung:
**26.06.2002 Patentblatt 2002/26**

(21) Anmeldenummer: **99904717.8**

(22) Anmeldetag: **12.01.1999**

(51) Int Cl.[7]: **G06F 17/00**, A61B 5/04

(86) Internationale Anmeldenummer:
**PCT/DE99/00080**

(87) Internationale Veröffentlichungsnummer:
**WO 99/35558 (15.07.1999 Gazette 1999/28)**

(54) **VERFAHREN UND VORRICHTUNG ZUR DARSTELLUNG UND ÜBERWACHUNG VON FUNKTIONSPARAMETERN EINES PHYSIOLOGISCHEN SYSTEMS**

METHOD AND DEVICE FOR REPRESENTING AND MONITORING FUNCTIONAL PARAMETERS OF A PHYSIOLOGICAL SYSTEM

PROCEDE ET DISPOSITIF DE REPRESENTATION ET DE SURVEILLANCE DE PARAMETRES FONCTIONS D'UN SYSTEME PHYSIOLOGIQUE

(84) Benannte Vertragsstaaten:
**AT CH DE DK ES FI FR GB IT LI SE**

(30) Priorität: **12.01.1998 DE 19801240**

(43) Veröffentlichungstag der Anmeldung:
**02.11.2000 Patentblatt 2000/44**

(73) Patentinhaber: **energy lab Beteiligungs GmbH 86159 Augsburg (DE)**

(72) Erfinder:
• **SOULA, Anatoli**
  **Moscow Region, 140003 (RU)**
• **KITACHINE, Youri**
  **Moscow Region,140003 (RU)**
• **GILLESSEN, Werner**
  **D-15344 Strausberg (DE)**

(74) Vertreter: **Reinhard - Skuhra - Weise & Partner**
**Postfach 44 01 51**
**80750 München (DE)**

(56) Entgegenhaltungen:
**US-A- 5 215 099**

• **MOURA L ET AL: "A microcomputer-based cardiac mapping system for recurrent ventricular tachycardia surgery" PROCEEDINGS OF COMPUTER IN CARDIOLOGY 1992 (CAT. NO.92CH3259-9), DURHAM, NC, USA, 11-14 OCT. 1992, Seiten 431-434, XP002107065 ISBN 0-8186-3552-5, 1992, Los Alamitos, CA, USA, IEEE Comput. Soc. Press, USA**
• **BRANHAM B H ET AL: "A system for accurate interactive 3-D display of cardiac electrical activity" PROCEEDINGS OF COMPUTER IN CARDIOLOGY 1992 (CAT. NO.92CH3259-9), DURHAM, NC, USA, 11-14 OCT. 1992, Seiten 335-338, XP002107066 ISBN 0-8186-3552-5, 1992, Los Alamitos, CA, USA, IEEE Comput. Soc. Press, USA**
• **YOUNG M R ET AL: "A real-time data acquisition system for the display of three dimensional cardiac activation maps" PROCEEDINGS OF COMPUTER IN CARDIOLOGY 1992 (CAT. NO.92CH3259-9), DURHAM, NC, USA, 11-14 OCT. 1992, Seiten 331-334, XP002107067 ISBN 0-8186-3552-5, 1992, Los Alamitos, CA, USA, IEEE Comput. Soc. Press, USA**
• **CALDERON A ET AL: "3-D mapping of body surface potentials" MEDICAL INFORMATICS, APRIL-JUNE 1987, UK, Bd. 12, Nr. 2, Seiten 125-135, XP002107068 ISSN 0307-7640**

**Beschreibung**

Die Erfindung betrifft ein Verfahren und eine Vorrichtung zur Darstellung und Überwachung von Funktionsparametern eines physiologischen Systems, insbesondere elektrokardiographischer Daten, die aus elektronischen Meßsignalen abgeleitet werden.

Seit der Entdeckung der Aktionsströme des Herzens im Jahre 1887, werden im Rahmen der Elektrokardiographie die ermittelten Daten registriert. Nachdem zunächst das Saitengalvanometer in Verbindung mit einem lichtempfindlich beschichteten Papierstreifen zum Nachweis der Aktionspotentiale eingesetzt wurde, werden seit mehreren Jahrzehnten schreibende Meßwerke verwendet. In diese Meßwerken führen Schreibmittel, vergleichbar einem Plotter, eine lineare Bewegung in Abhängigkeit der verstärkten Meßwerte aus, wobei ein Papierstreifen senkrecht zu der Bewegung der Schreibmittel unter diesen entlanggeführt wird.

Auf diese Weise entsteht eine Kurve, die einen typischen, sich periodisch wiederholenden Verlauf aufweist. Anhand dieser Kurve ist es einem ausgebildeten und geschulten Kardiologen möglich, Änderungen der Reizbildung, Rhythmusstörungen oder Schädigungen des Herzmuskels zu erkennen. Nachteilig an dieser Art der Darstellung ist, daß der Informationsgehalt der Messungen, insbesondere bei geringen Veränderungen nur schwer zu erkennen ist und daß zu der Deutung der Kurven eine spezielle Ausbildung und eine große Erfahrung notwendig ist. Weiterhin bedarf es für eine sorgfältige Auswertung eines Elektrokardiogrammes einer nicht unerheblichen Zeitdauer.

In der weiteren Entwicklung wurde die Kurve auf einem Bildschirm dargestellt, was eine wesentlich verfeinerte Darstellung ermöglichte, da der. Elektronenstrahl eine geringere Massenträgheit aufweist als das Schreibgerät und somit nahezu verzögerungsfrei arbeitet. Nachteilig daran ist die kurze Darstellung der Kurve und die eingeschränkte Auflösung auf dem Bildschirm.

Aus der zweidimensionalen Darstellung wurde die Vektorkardiographie entwickelt, in der die Aktionsströme der Herzmuskelfasern dergestalt aufgezeichnet werden, daß Schleifen in den drei Ebenen des. Raumes entstehen, wobei die Interpretation der Raumkurven lediglich von geschultem Fachpersonal durchgeführt werden kann.

Aus der US-5,215,099 ist eine Vorrichtung und ein Verfahren zur Voraussage von Herzrhythmusstörungen bekannt, bei der eine Vielzahl von EKG-Messungen des QRS-Bereiches gemittelt werden und untypische Wellenformen aussortiert werden. Die gemittelten Werte der EKG-Messungen werden anschließend digitalisiert, die Segmente zueinander zeitlich verschoben, analysiert und mittels einer Fouriertransformtion transformiert. Die transformierten Daten werden in einer dreidimensionalen Form ausgedruckt, wobei bei einer Übereinstimmung der zeitlich verschobenen Segmente untereinander auf ein geringes Infarktrisiko geschlossen werden kann.

In den Aufsätzen "A microcomputer-based cardiac mapping system for recurrent ventricular tachycardia surgery"; Moura et al., "A system for accurate interactive 3-D display of cardiac electrical activity"; Branham et al., "A real-time data acquisition system for the display of three dimensional cardiac activation maps"; Young et al. und "3-D mapping of body surface potentials"; Calderon et al. werden verschiedene Methoden der Darstellung von Oberflächenpotentialen beschrieben, die zwar eine ortsgenaue Zuordnung der Meßstellen zulassen, deren Auswertung jedoch Erfahrung erfordert.

Aufgabe der vorliegenden Erfindung ist es, ein Verfahren und eine Vorrichtung bereitzustellen, mit der schnell und für jedermann nutzbar eine Analyse der Veränderung von Prozeßzuständen und die Prognose künftiger Zustände ermöglicht wird.

Erfindungsgemäß wird diese Aufgabe durch ein Verfahren gemäß Anspruch 1 und eine Vorrichtung gemäß Anspruch 17 gelöst.

Das Wort Informationsmikroskop veranschaulicht sowohl das erfindungsgemäße Verfahren als auch die Vorrichtung zu dessen Durchführung, da vorliegende Informationen, die einem Betrachter ohne. weiteres nicht zugänglich sind, der menschlichen Wahrnehmung zugeführt werden. Bei einem Lichtmikroskop werden kleinste Strukturen der Materie durch eine Brechung der Lichtwellen in einem Linsensystem sichtbar. Das Informationsmikroskop bereitet Funktionsparameter eines physiologischen Systems dergestalt auf, daß diese überhaupt der allgemeinen Wahrnehmung zugänglich gemacht und daß kleinste Veränderungen deutlich werden.

Die Deutung von komplexen Informationen, wie sie beispielsweise in einem EKG enthalten sind, wird durch das erfindungsgemäße Verfahren wesentlich vereinfacht und beschleunigt. Durch die sehr kurze Meßdauer und die einfache Anwendung ist eine häufige Datenaufnahme möglich, so daß eine Veränderung eines physiologischen Systems durch Messung in kurzen Abständen über einen langen Zeitraum beobachtet werden kann.

Die kurze Auswertedauer und die einfache Interpretierbarkeit der aufbereiteten Meßwerte machen auch sehr kleine Veränderungen offensichtlich. In Zusammenhang mit der Möglichkeit einer qualitativen Einschätzung der dargestellten Meßwerte sind Prognosen über das untersuchte physiologische System möglich.

Durch die Umwandlung in ein graphisches Portrait wird eine individuelle Abbildung des untersuchten Systems angefertigt, anhand dessen eine Identifizierung, wie bei einer Photographie, möglich ist.

Mit Vorteil ist das graphische Portrait nach Art eines dreidimensionalen topologischen Modells aufgebaut, da durch diese Darstellung, die der natürlichen Wahrnehmung der Umwelt am nächsten kommt, die höchste Informationsdichte übermittelt werden kann. So können auch komplexe Meßwerte anschaulich und leicht zugänglich dargestellt werden. Ebenso werden kleine Abweichungen, die in einem zweidimensionalen Diagramm nur schwer zu identifizieren wären, durch diese Art der Darstellung aufgrund der größeren Informationsmenge deutlicher wahrnehmbar. Ähnlich einer Landkarte, bei der die topologischen Gegebenheiten durch eine entsprechende Gestaltung sichtbar werden, vermittelt das dreidimensionale Modell eine Ansicht der Funktionsparameter mit hoher Anschaulichkeit und Informationsdichte.

Die Digitalisierung der Daten erlaubt eine Zuordnung von bisher in einer Längserstreckung aufgetragenen Bereichen zu räumlichen Koordinaten, wodurch eine dreidimensionale Wirkung erzielt wird. Ausgehend von einer signifikanten Meßgröße werden bestimmte Abschnitte des Meßwerteverlaufs bestimmten Raumarealen zugeordnet. Das analoge Signal wird digitalisiert und der Wert wird in Abhängigkeit seines zeitlichen Auftretens als Stützstelle zur Bildung einer weitgehend geschlossenen Oberfläche herangezogen. Zusätzlich werden die einzelnen Stützstellen mit einem Farbcode hinterlegt, der sowohl Crominanz als auch Luminanz umfaßt. Diese Farbcodehinterlegung erfolgt auf der Basis der Meßwerte jeweils eines Analysezyklus und ermöglicht eine qualitative Einordnung anhand der Farb- und Helligkeitswerte des Bildes.

Die Festlegung des Analysezyklus erfolgt vorteilhafterweise durch die Ermittlung des zeitlichen Abstandes zumindest zweier sich wiederholender signifikanter Größen, wobei die Größen abhängig von den physiologischen Gegebenheiten bzw. von dem untersuchten Körperteil ist. Bei kardiologischen Untersuchungen bietet sich der Abstand zweier R-Impulse als die Länge eines Analysezyklusses an, da dieser leicht zu bestimmen ist und in der Regel eine ausreichende Flankensteilheit aufweist. Der Analysezyklus wird dabei dergestalt festgelegt, daß die signifikanten Aktionspotentiale des Herzmuskels erfaßt werden, also daß der gesamte Bereich von P bis T abgedeckt ist.

Um die Genauigkeit und Aussagekraft der Messung sowie die Präzision der Darstellung zu erhöhen, ist eine Meßdauer von Vorteil, die ein Vielfaches eines Analysezyklus umfaßt. Da die diskreten Werte der digitalisierten Messung entsprechend ihrer Reihenfolge bestimmten Gebieten innerhalb der bildhaften Darstellung zugeordnet werden, ist eine Messung über einen Zeitintervall günstig, der den gesamten Bereich der zu berücksichtigenden Werte beinhaltet.

Es ist insbesondere von Vorteil, daß die Zuordnung von Farbcodes durch eine Verknüpfung von empirisch ermittelten Vergleichsdaten errechnet wird. Auf der Basis von Daten, die in einem elektronischen Speicher hinterlegt und aus diesem abgerufen werden, werden den Stützstellen Informationen bezüglich der Farbe und der Helligkeit zugeordnet. Die Zuordnung erfolgt in Abhängigkeit von den Abweichungen der gemessenen Werte zu den Vergleichsdaten. Je nach Grad der Abweichung und der Position innerhalb der Kurve wird den Meßwerten ein entsprechender Farb- und Helligkeitswert zugeordnet, so daß ein Bild entsteht, das aufgrund der Form, Farbe und Helligkeit eine einfache Diagnose zuläßt, weil die Art der Informationsdarstellung auf der Grundlage medizinischer Vergleichsdaten verändert wurde und eine einfache Zuordnung auch kleinster Abweichungen erlaubt.

Damit keine offensichtlichen Störsignale weiterverarbeitet werden und dadurch den Bearbeitungsaufwand verlängern und die Genauigkeit der Anzeige verringern, ist in einer Weiterbildung der Erfindung eine Auswahl der auszuwertenden Analysezyklen nach der Berechnung der Autokorrelationsfunktion und eines Vergleiches mit empirisch ermittelten Vergleichsdaten vorgesehen.

Um die aufgenommenen Daten eines Analysezyklusses zu analysieren, werden sie durch einen Hochpaßfilter geleitet, digitalisiert und die so erhaltenen Werte werden von den ungefilterten Daten, die ebenfalls digitalisiert wurden, subtrahiert. Die so gewonnenen Daten werden entsprechend ihrem Wert zu Gruppen oder sog. Clustern zusammengefaßt, so daß bei Betrachtung mehrerer Analysezyklen in einem Zeitintervall jeweils Gruppen mit gleichem Wert in einer Matrix benachbart angeordnet werden. Durch das Gruppieren der Werte werden die zunächst regellos verteilten Kleinschwankungen geordnet und aus dem, auf den ersten Blick zufälligen Kleinschwankungen eine reguläre Struktur mit einigen konstanten Merkmalen heraus. Die Reihenfolge der Gruppierung wird für jedes physiologische System, also für jeden Patienten, individuell gespeichert.

Vorteilhafterweise wird die Reihenfolge der Gruppierung im Verlauf einer Referenzmessung ermittelt und auf nachfolgende Analysezyklen angewendet. Ergibt die erneute Anwendung der Reihenfolge der Umgruppierung eine veränderte Struktur, kann daraus auf Veränderungen des physiologischen Systems geschlossen werden.

In einer vorteilhaften Ausgestaltung der Erfindung werden sämtliche Analysezyklen eines Zeitintervalles bei der Bildberechnung berücksichtigt, da sich durch eine Vermehrung der Stützstellen die Auflösung erhöht. Die vorhandenen Abweichungen bewirken eine Streuung der Stützstellen, so daß sich eine Clusterbildung bei der Auswertung mehrerer Analysezyklen ergibt.

Besonders für die Anwendung im persönlichen Bereich ist die Ermittlung von Aktionspotentialen bei Herzuntersuchungen nach einer standardisierten 3-Punkt-Ableitung angebracht, da auf diese Art und Weise auch bei weniger empfindlichen Meßwertaufnehmern eine ausreichende Genauigkeit der Messung erreicht wird. Für eine Anwendung in kardiologischen Diagnostikbereich sind auch andere Ableitungen, beispielsweise die 2- oder 6-Punkte- Ableitung vorgesehen, wobei die letztere Methode die aussagekräftigsten Informationen liefert, aufgrund derer der Fachmann qualitative und quantitative Diagnosen einfach und schnell erstellen kann.

In einer Weiterbildung der Erfindung erfolgt die Eliminierung von Störgrößen durch eine Korrelation der Meßwerte mehrerer Analysezyklen. Da das Auftreten von Störgrößen bei Meßwertaufnahmen zufällig ist, die eigentlichen Meßwerte jedoch untereinander korrelieren, werden die Störsignale durch eine Korrelation herausgefiltert, so daß auch kleine Abweichungen in der Bilddarstellung erfaßt werden, die in der herkömmlichen Darstellung im Rauschen unerkannt geblieben wären.

In einer Ausgestaltung der Erfindung werden die aufgenommenen Meßwerte über Online-Datenleitungen beispielsweise an ein Diagnosezentrum weitergeleitet, wo eine Auswertung und Archivierung der Daten erfolgt. Die zu untersuchende Person müßte nicht mehr vor Ort untersucht werden, sondern könnte in der gewohnten Umgebung zu einer passenden Zeit die Daten aufnehmen, die dann von einem Fachpersonal ausgewertet werden.

Als Weiterbildung der Erfindung ist eine Hinterlegung der Bilddarstellung mit einer Therapieempfehlung vorgesehen, so daß die eigene Deutung der Meßergebnisse mit einer auf empirischem wege ermittelten Empfehlung untermauert werden.

Eine Vorrichtung zur Durchführung des Verfahrens nach Anspruch 1 mit einem Informationsmikroskop weist Vorrichtungen zur Datenaufnahme und zur Umwandlung in elektrische Signale sowie eine Vorrichtung zur Festlegung eines Analysezyklus auf. Weiterhin ist ein Analog-Digitalwandler vorgesehen, der die aufgenommenen Daten digitalisiert und einer Speichereinheit zuführt. In einer daran angeschlossenen Recheneinheit mit einer Auswertesoftware werden die Daten entsprechend ihrer Abweichung von den in der Speichereinheit hinterlegten Daten mit Crominanz- und Luminanzwerten versehen und einem dreidimensionalen Koordinatensystem zugeordnet. Die einzelnen Werte der digitalisierten Signale bilden Stützstellen, die zu einer geschlossenen Fläche verbunden werden. In einer Steuerungseinheit werden die ermittelten Bildpunkte für die jeweilige Ausgabeeinrichtung aufbereitet. Die Auswerte- oder Ausgabeeinrichtung übermittelt die Bildinformationen in Gestalt eines Farbbildes oder eines Farbausdruckes.

In einer Ausgestaltung des Informationsmikroskopes sind die Vorrichtungen zur Datenaufnahme als Elektroden, insbesondere als Haft- oder Klemmelektroden ausgebildet, da auf diese Weise leicht Informationen über Aktionspotentiale von Muskelaktivitäten gewonnen werden können. Gerade die Klemmelektroden sind leicht zu handhaben und bieten auch einem Laien die Möglichkeit, Daten über ein physiologisches System zu gewinnen. Je nach Art der aufgenommenen Größe sind Drucksensoren, Durchflußmengenmesser oder optische Sensoren einzusetzen, wobei diese auch in Kombination verwendet werden können. Grundsätzlich sind alle Meßwertaufnehmer geeignet, die Aussagen über physiologische Systeme ermitteln, wobei die Umwandlung in elektrische Signale in Abhängigkeit von dem aufgenommenen Meßwert beispielsweise in einem Druckumformer oder einen photoelektrischen Wandler erfolgt.

Da die elektrischen Signale häufig eine zu geringe Amplitude aufweisen, ist vorteilhafterweise ein Verstärker vorgesehen, mit dem ein ausreichend kräftiges Signal bereitgestellt werden kann.

Für eine schnelle Berechnung des topologischen Modells im Informationsmikroskop sind in der Speichereinheit empirisch ermittelte Vergleichsdaten abgelegt, die eine Zuordnung der ermittelten Werte entweder zu vorherigen Messungen oder zu krankhaften Veränderungen erlauben, wobei die Speichereinheit vorteilhafterweise mit der Recheneinheit, die vorzugsweise als Computer ausgeführt ist, verbunden ist.

Für eine unkomplizierte Bereitstellung des topologischen Modells ist insbesondere die Ausgabe an einen Monitor vorteilhaft, wobei ein Farbmonitor die Aussagekraft des Modells der Funktionsparameter erhöht. Weiterhin sind Drucker, insbesondere Farbdrucker, oder andere bilderzeugenden Medien wie Bildbelichter oder Projektoren als Ausgabemedium vorgesehen.

In einer Ausgestaltung der erfindungsgemäßen Vorrichtung ist eine Schnittstelle zur externen Übermittlung von Daten vorgesehen, damit eine Beurteilung der gemessenen Werte beispielsweise in einem Diagnosezentrum erfolgen kann, ohne daß die untersuchte Person anwesend sein muß. Insbesondere bei kontinuierlichen Überwachungen ist diese Untersuchungsmethode von Vorteil.

Anhand der Zeichnungen werden nachfolgend Ausführungsbeispiele der Erfindung näher erläutert. Es zeigen:

Fig. 1 -     eine Darstellung eines Normalelektrokardiogrammes;

Fig. 2 -     eine Darstellung eines Patienten-EKG;

Fig. 3 -     ein Blockschaltbild des Informationsmikroskopes;

Fig. 4 -     ein Schema der Analysezyklenbildung;

Fig. 4a -     eine Abfolge mehrerer EKG-Signale in einem Zeitintervall;

Fig. 4b -     eine Darstellung der Digitalisierung eines EKG-Signals;

Fig. 5 -     eine Prinzipdarstellung der Umgruppierung von Matritzenwerten;

Fig. 6 -      eine Darstellung unterschiedlicher Phasen der Umgruppierung der Differenzmatrix;

Fig. 7 und 8 -      Blockschaltbilder der Signalverarbeitung;

Fig. 9 -      eine Zusammensetzung verschiedener Farbspektren in Abhängigkeit von der Abszissenwerte des EKG-Signals;

Fig. 10 und 11-      Blockschaltbilder der Signalverarbeitung;

Fig. 12 -      eine Prinzipdarstellung der Zuordnung von Stützstellen zu Raumkoordinaten;

Fig. 13 -      eine beispielhafte Darstellung des bearbeiteten EKG-Signals, sowie

Fig. 14 -      eine Prinzipdarstellung der Entsprechungen zwischen den Bereichen eines EKG und des erstellten topologischen Modells.

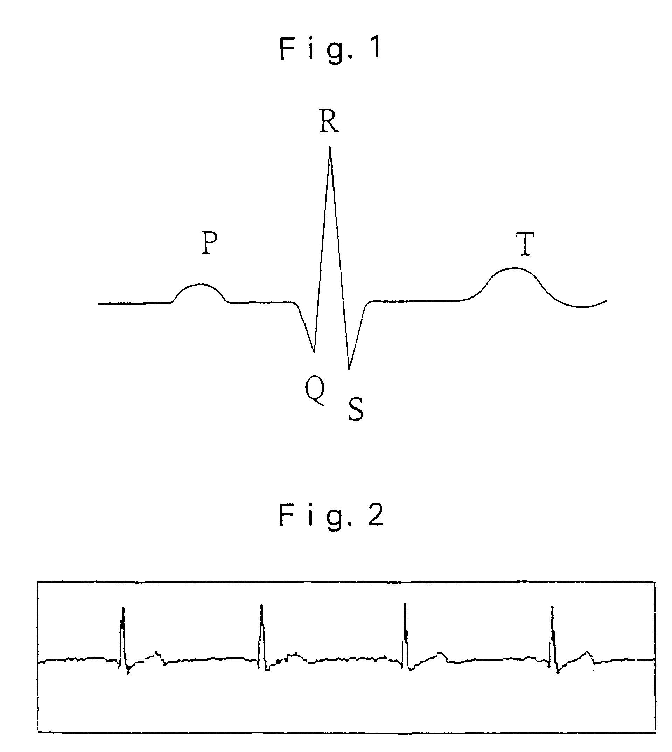

Figur 1 zeigt ein herkömmliches Elektrokardiogramm (EKG) in seiner zweidimensionalen Darstellung, in der auf der Abszisse die Zeitachse und auf der Ordinate die Aktionspotentiale aufgetragen sind. Ein solches Normal-EKG kann in vier Bereiche eingeteilt werden, deren Beginn und Ende von P bis T mit Buchstaben bezeichnet werden. Der Bereich von P bis Q entspricht der Kontraktion der Vorhöfe, von Q bis T der Kontraktion der Kammern des Herzens. Die QRS-Gruppe entspricht der Erregungsausbreitung in den Herzkammern und die T-Zacke kommt durch die örtlich verschieden ablaufende Repolarisation zustande. Die QRS-Gruppe, die ST-Strecke und die T-Zacke zusammen bilden die Kammergruppe des EKG.

In Figur 2 ist anhand eines Patienten-EKG zu erkennen, daß der Kurvenverlauf dem eines Normal-EKG entspricht, wobei allerdings keine geraden Verbindungen zwischen den jeweiligen Punkten auftreten, sondern entsprechend den Verläufen der Aktionspotentiale ständig relativ kleine Ausschläge zu registrieren sind. Ein solches EKG ist auch für einen Fachmann nur mit großer Erfahrung zu deuten. Insbesondere die kleinen Veränderungen von Herzschlag zu Herzschlag sind nicht augenfällig und werden leicht übersehen, obwohl die Informationen erfaßt worden sind.

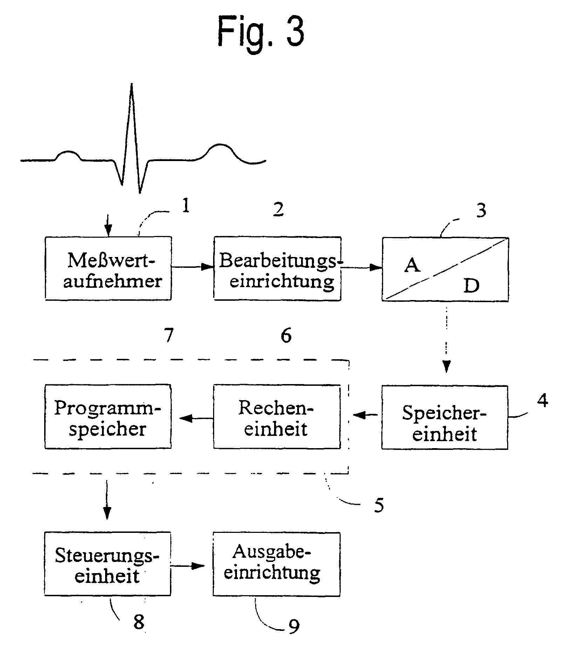

Das Blockschaltbild in Figur 3 veranschaulicht die Vorgehensweise bei einer EKG-Messung. Die zu untersuchende Person befestigt zwei standardisierte Elektroden an den üblichen Meßpunkten (Hand- und/oder Fußgelenke) und eine neutrale Elektrode am Ohrläppchen. Über eine nicht dargestellte Auslöseeinrichtung wird die Meßwertaufnahme für einen bestimmten Zeitintervall durchgeführt. Die Aktionsströme werden in einem Meßwertaufnehmer 1 verstärkt und an eine Bearbeitungseinrichtung 2 weitergeleitet. Falls die Meßwerte nicht in Form von elektrischen Signalen vorliegen, findet zunächst eine Signalumwandlung statt.

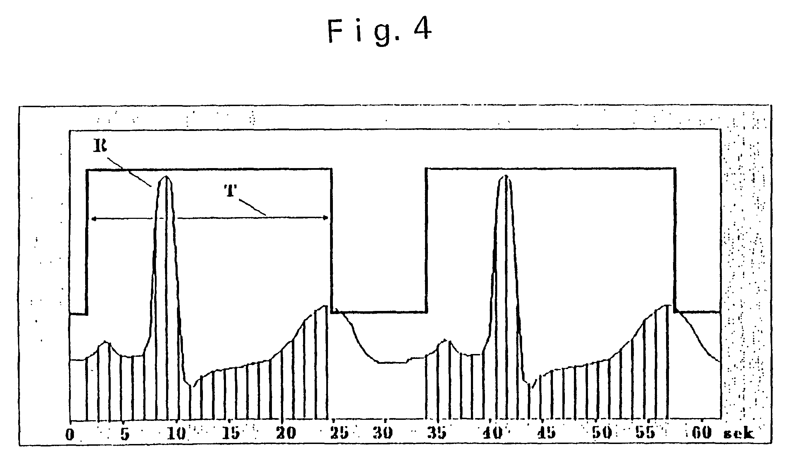

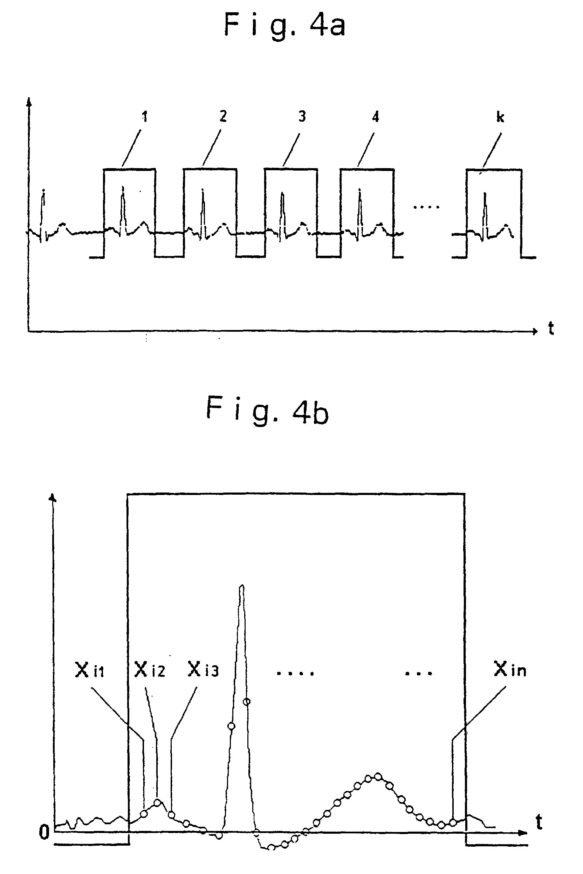

In der Bearbeitungseinrichtung 2 wird die Länge des Analysezyklus festgelegt, die sich nach der Dauer zweier Herzschläge richtet. In der Figur 4 wird eine solche Festlegung schematisch dargestellt, bei der ausgehend von dem Abstand zweier aufeinanderfolgender R-Zacken für die Analysezykluslänge T ein verkürztes Intervall bestimmt wird. Als Anhaltswert für T sind 80-90% des R-R-Abstandes vorgesehen, womit die QRST-Gruppe erfaßt wird. Der Zeitraum T umfaßt also die gesamte Kammergruppe des EKG und in einem Analog/Digitalwandler 3 werden die analog vorliegenden Signale digitalisiert, so daß jeweils eine bestimmte Anzahl von n Werten pro Analysezyklus zur Verfügung steht. Da der Analysezyklus von einer signifikanten Größe aus berechnet wird, in dem gewählten Beispiel von der R-Zacke, lassen sich die jeweiligen digitalisierten Werte einem bestimmten Bereich des EKG zuordnen.

Das Signal ist in dem dargestellten Beispiel aus Gründen der Darstellbarkeit in 25 Werte unterteilt, wobei selbstverständlich auch eine höhere Auflösung vorgenommen werden kann. Die Anzahl der Zeiträume T, also der innerhalb eines Meßzeitraumes aufgenommenen Analysezyklen, wird mit dem Index i versehen. Bei einem Analysezyklus von 1 Minute und einer Herzfrequenz von einem Schlag pro Sekunde läuft der Index i von 1 bis 60. Die digitalisierten Werte werden einer Speichereinheit 4 zugeführt, an die eine Auswerteeinheit 5, bestehend aus einer Recheneinheit 6 mit einer Auswertesoftware und einem Programmspeicher 7, angeschlossen ist.

In der Figur 4a ist eine EKG-Messung über einen längeren Zeitraum aufgetragen, wobei k-mal ein Fenster von der Länge T mit der QRST-Gruppe gebildet wird. In der Figur 4b ist detailliert dargestellt, wie die Ermittlung der digitalisierten Werte $X_{ij}$ erfolgt, wobei der Index i die jeweilige QRST-Gruppe bezeichnet und von 1 bis k läuft und der Index j von 1 bis n läuft und den jeweiligen Meßpunkt bezeichnet. Jedem der Meßpunkte j wird ein X-Wert zugeordnet, so daß eine Matrix $X_{ij}$ mit k Zeilen und n Spalten entsteht.

In jeder QRST-Gruppe wird mittels eines Hochpaßfilters eine Beseitigung der Kleinschwankungen vorgenommen, wobei als Kleinschwankungen Veränderungen im Bereich von 0,5-1,5% von dem Wert der R-Zacke angesehen werden. Die Hochpaßfilterung ergibt nach der Digitalisierung auch eine k x n -Matrix, allerdings ohne Kleinschwankungen. Diese Matrix $Xf_{ij}$ wird von der Matrix $X_{ij}$ subtrahiert, so daß eine Differenzmatrix $Xd_{ij}$ entsteht, in der nur die Kleinschwankungen enthalten sind.

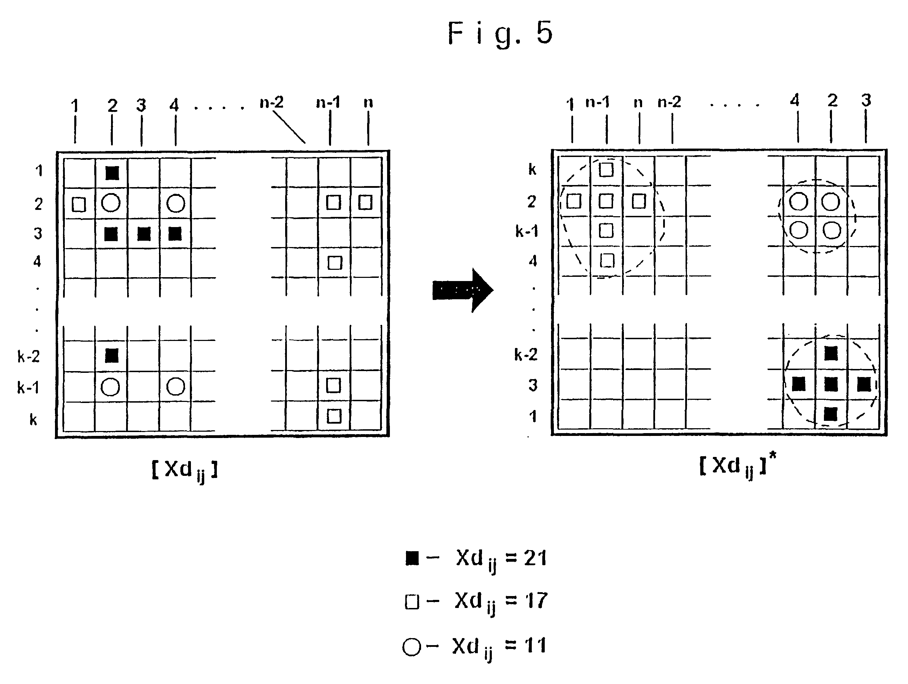

Die ermittelte Differenzmatrix $Xd_{ij}$ wird dergestalt transponiert, daß die nahe beieinanderliegenden Werte zu Gruppen oder Cluster zusammengefaßt werden. In der Figur 5 ist eine solche Umgruppierung der Matrix dargestellt, wobei die linke Matrix die ermittelte Differenzmatrix darstellt und die rechte Matrix die fertig umgruppierte Matrix. Die verschiedenen Symbole innerhalb der Matrix $Xd_{ij}$ repräsentieren jeweils gleiche Werte, hier die Werte 11, 17 und 21. Die Matrix $Xd_{ij}$ wird durch aufeinanderfolgende Transpositionen dergestalt umgeordnet, daß die jeweiligen Werte (hier durch Symbole verdeutlicht) möglichst zu Gruppen zusammengefaßt beieinanderstehen, so wie in der rechten Matrix $Xd_{ij}^*$ in Figur 5 dargestellt. Die jeweiligen Werte bilden sogenannte Cluster und der Vorgang der Umgruppierung oder das Transponieren ist anhand der veränderten Spalten- und Zeilenindexierung leicht zu erkennen. Die Reihenfolge der Transpositionen ist für jede Person verschieden und wird im Verlauf einer Referenzmessung ermittelt und als individuelle Funktion gespeichert.

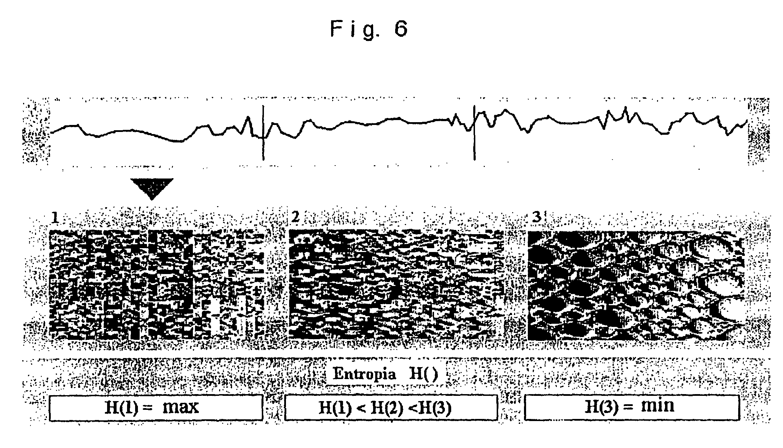

Die Figur 6 zeigt das Ergebnis der Umstellung der Differenzmatrix anhand der Darstellungen 1 bis 3, wobei die Darstellung die Ausgangsdifferenzmatrix abbildet, also das Signal vor der Umgruppierung. In der Darstellung 2 sind die Hälfte der Umstellungen vorgenommen worden, so daß bereits erste Cluster sich abzeichnen. Die Darstellung 3 in der Figur 6 zeigt die endgültige Variante der Transposition, bei der die jeweiligen Werte optimal zu Clustern zusammengestellt wurden. Die Transposition der Werte führt zu einer erhöhten Ordnung innerhalb der Differenzmatrix, so daß man auch von einer Verringerung der "Informationsentropie" durch die Bildung der Cluster sprechen kann. Ist in der Darstellung 1 noch ein ungeordneter Zustand der Werte vorhanden, H(1) nimmt also einen maximalen Wert ein, verringert sich diese Unordnung mit zunehmender Umgruppierung, bis sie schließlich minimal wird (H(3) = min).

Durch die Transposition der Differenzmatrix $Xd_{ij}$ zur Matrix $Xd_{ij}^*$, wird aus den anfänglichen, auf den ersten Blick zufälligen Kleinschwankungen eine innere Struktur erkennbar. Ist diese Struktur konstant, daß heißt, treten an den jeweiligen Stellen die gleichen Schwankungen auf, wird sie sich bei der nächsten Anwendung der ermittelten Reihenfolge der Transpositionen der Matrix $Xd_{ij}$ wiederholen.

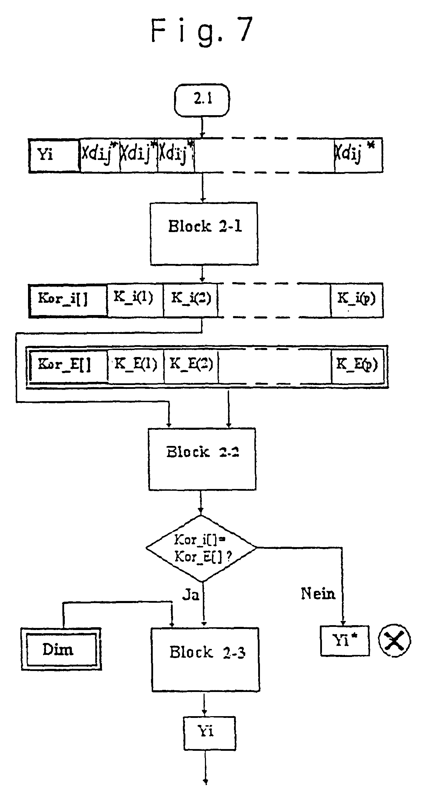

Die Verarbeitung mit der Auswertesoftware arbeitet nach einem als Blockschaltbild in der Figur 7 dargestellten Prinzip, wobei mittels einer weiteren Prozedur die unstabilen, für die Analyse der aufgenommenen Daten ungeeigneten Schwankungen entfernt werden. Das Signal $Yi= Xdij^*$ (i=1, ..., k; j=1,...,n), welches von den anderen Werten $Xdi1^*$, $Xdi2^*$,..., $Xdin^*$ abhängt, wird an die Einheit 2-1 übermittelt, in dem die Autokorrelationsfunktion Fkor_i der Datengruppe errechnet wird. Diese Funktion zeigt an, ob eine Größe von einem eigenen Wert zu einem früheren Zeitpunkt abhängt, also ob sie z.B. versteckte Periodizitäten enthält. Scharfe Maxima oder Minima werden dabei an denjenigen Stellen angezeigt, die Perioden oder Antiperioden des Vorgangs entsprechen. Diese Funktion wird an den ersten der zwei Eingänge des Blockes 2-2 weitergeleitet.

Der zweite Eingang wird mit der Funktion Fkor_E beaufschlagt, die in dem Programmspeicher 7 hinterlegt ist und als das Normal zum Vergleich herangezogen wird. Die Funktion Fkor_E ist eine als Referenz aufgenommene, bezüglich der Autokorrelation bearbeitete Messung, die für jeden zu Untersuchenden erstellt wird. Auf der Grundlage dieser Referenzmessung werden in der Einheit 2-2 werden die Funktionen Fkor_i und Fkor_E nach definierten Kriterien miteinander verglichen. Da die Signifikanz der Veränderungen des physiologischen Systems anhand von Kleinstschwankungen innerhalb der aufgenommenen Signale deutlich wird, werden diejenigen Signale herausgefiltert, die eine zu große Differenz zu der Referenzmessung aufweisen. Auf diese Weise werden die beiden Funktionen nur innerhalb eines vorab festgelegten Bereiches miteinander verglichen. Bei nichtvorhandener Ähnlichkeit, also bei zu großen Abweichungen der untersuchten Signale von der Referenzmessung, wird die Weiterverarbeitung des Signals gestoppt.

Liegt Ähnlichkeit nach den vorgegebenen Kriterien vor, wird das Signal Yi in der Einheit 2-3 weiterverarbeitet. An einem zweiten Eingang der Einheit 2-3 wird eine Vergrößerungsfunktion Dim mit einem Vergrößerungsfaktor m angelegt, der ebenfalls in dem Programmspeicher 7 abgelegt ist. Aus dem Signal Yi und dem Vergrößerungsfaktor wird ein auf einen festgelegten Bereich verstärktes, also ein normiertes Signal erstellt. Dieses verstärkte Signal wird anschließend an die Einheit 2-4 weitergeleitet.

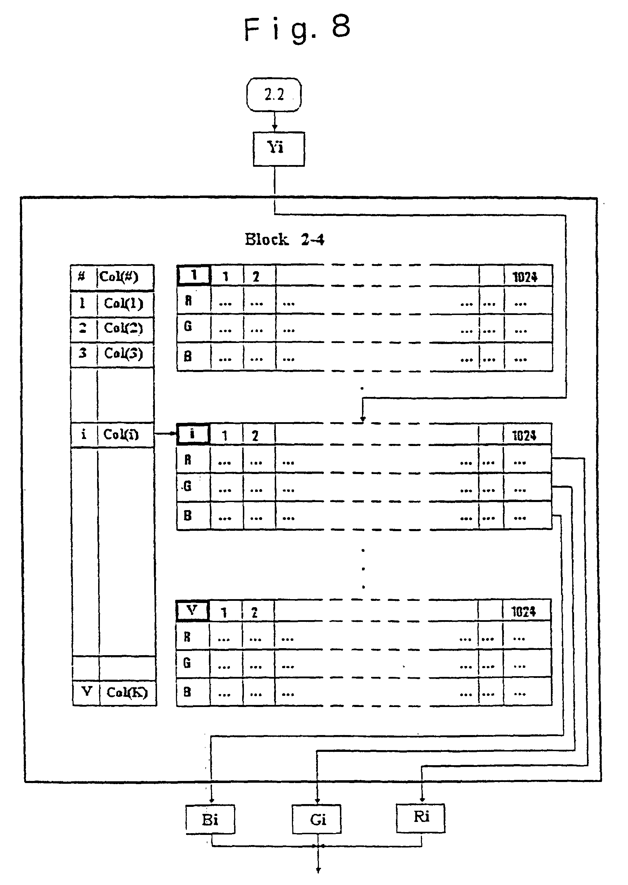

In der Figur 8 ist anhand eines Blockschaltbildes die Zuordnung des Farbcodes dargestellt. Das normierte Signal Yi wird in einem weiteren Schritt mit Farbanteilen verknüpft, die in Tabellen abgelegt sind. Diese Tabellen enthalten n Spalten und drei Zeilen, wobei die letzteren einem roten (R), grünen (G) und blauen (B) Farbanteil entsprechen. In dem gewählten Beispiel liegen 1024 Werte des Signals Yi vor. Jeder der 1024 Werte repräsentiert den Wert der Abweichung an einer bestimmten Stelle des Zeitbereiches T und kann somit einem bestimmten Bereich der Herzmuskelaktivität zugeordnet werden, beispielsweise dem Bereich der S-Zakke.

Jeden dieser 1024 Werte wird dann ein Farbwert zugeordnet, der aus einem bestimmten Bereich eines Farbspektrums ausgewählt wird. Die Werte Col(i) in den Farbtabellen werden dem jeweiligen Signal Yi zugeordnet, so daß am Ausgang der Einheit 2-4 drei sekundäre Farbcodes Ri, Gi, und Bi dem Signal Yi zugeordnet sind. Dies bedeutet, daß jedem Punkt der Matrix $Xd_{ij}^*$ ein eigener Farbwert zugeordnet wird; für jeden der 1024 Werte wird aus einem für den jeweiligen Bereich des Signals zugeordneten Farbsprektrum ein Farbwert aus den Anteilen R (Rot), G (Gelb) und B (Blau) ermittelt.

Die Auswahl aus diesem Farbspektrum erfolgt in Abhängigkeit von der Abweichung zur Referenzkurve; d.h. ein Farbwert variiert, wenn die Abweichung sich verändert. Die Farbwerte sind in Tabellenform in dem Programmspeicher 7 abgelegt und werden dem jeweiligen Wert zugeordnet. Durch eine entsprechende Farbauswahl, einen Spektralverlauf und eine angepaßte Auflösung wird eine zuverlässige Unterscheidung bei kleinsten Differenzen ermöglicht und so eine Empfindlichkeitserhöhung bei der Erkennung von kleinsten Veränderungen bewirkt.

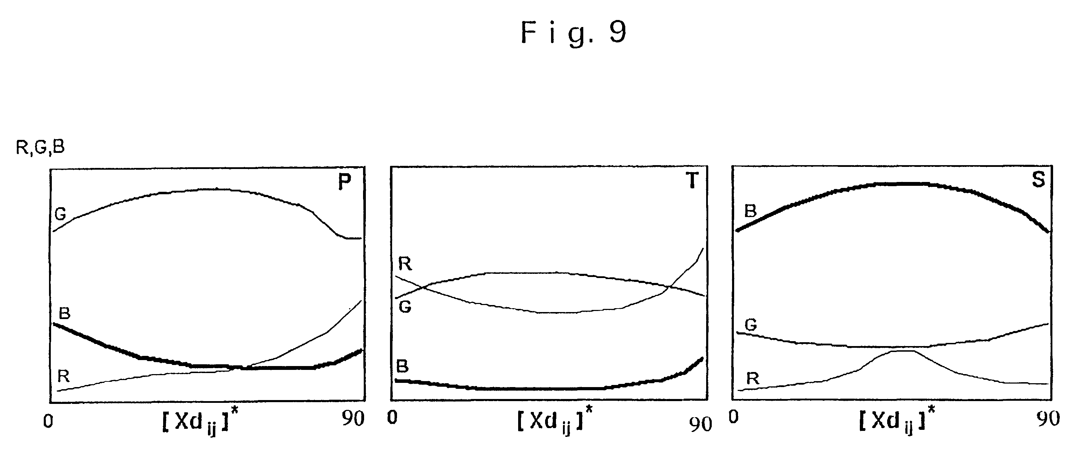

Figur 9 zeigt einen schematischen Verlauf von drei Farbspektren P, T und S, die jeweils dem gleichbenannten Bereich des EKG-Signals zugeordnet werden; beispielsweise werden dem P-Bereich die Farbe Grün, der T-Welle die Farbe Gelb und der S-Zacke die Farbe Blau zugeordnet. Über der Abszisse ist der jeweilige Verlauf der Farbanteile Gelb, Rot und Blau aufgetragen, wobei die Auswahl der Abszissenwerte in Abhängigkeit von der Größe des Signals $Xd_{ij}{}^*$ erfolgt. Ist beispielsweise keine Abweichung für den Bereich P nicht feststellbar, wird ein Farbspektrum in der Zusammenstellung von 0 ausgewählt. Bei einem sehr großen Wert für die Abweichung an der Stelle P ist ein Wert von 90 vorgesehen. Bei einer größeren Auflösung sind entsprechend andere Schrittweiten zu wählen.

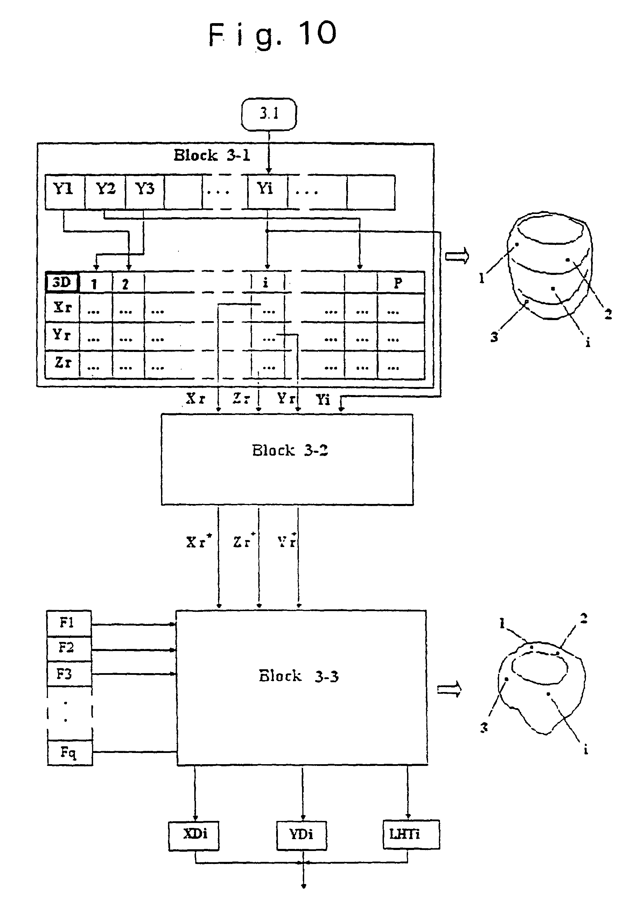

Die Weiterverarbeitung des nunmehr mit einem Farbcode versehenen Signals Yi in der Einheit 3-1 ist in der Figur 10 dargestellt. Die Einheit 3-1 enthält eine in dem Programmspeicher 7 abgelegte 3D-Tabelle mit drei Zeilen, die jeweils einer Raumdimension X, Y und Z entsprechen, und p Spalten (p<=K, mit K=Anzahl der Analysezyklen. Das mit dem Farbcode versehene Signal Yi wird mit den Komponenten Xr, Yr und Zr verknüpft, die die Ausgangskoordinaten für das Signal Yi in einem simulierten Raum darstellen.

Auch hier werden analog zu dem beschriebenen Verfahren der Farbcodezuordnung bestimmten Bereichen des EKG-Signals bestimmte Raumareale eines vorab festgelegten Grundmodells oder Grundkörpers zugeordnet. Je nach der Größe des Wertes Yi wird ein von dem Grundmodell abweichender Wert für die Raumkoordinaten Xr, Yr und Zr zugeordnet. Durch die von dem Grundmodell abweichenden Raumkoordinaten wird ein individuelles Bild erzeugt, daß die Kleinstschwankungen des aufgenommenen Signals wiedergibt. Es wird somit ein Portrait des untersuchten Herzens erstellt, das die Veränderungen des EKG-Signals eines Patienten als individuelle Darstellung wiedergibt, die in Form und Farbe, je nach den gemessenen Veränderungen, variiert.

Die Koordinaten Xr, Yr und Zr werden an drei Eingänge der Einheit 3-2 weitergeleitet, an deren vierten Eingang das Signal Yi anliegt. Aus diesen Größen werden, analog zu dem bei der Farbzuordnung beschriebenen Verfahren, die Koordinaten Xr*, Yr* und Zr* gebildet:

$$Xr^* = Xr + F\_X(Yi)$$

$$Yr^* = Yr + F\_Y(Yi)$$

$$Zr^* = Zr + F\_Z(Yi)$$

Die so ermittelten Koordinaten Xr*, Yr* und Zr* werden in der Einheit 3-3 auf eine Fläche projiziert, so daß die 3D-Animation auf einem Bildschirm oder einem Drucker ausgebbar wird. Bei entsprechender Geräteausstattung ist auch eine bewegliche Darstellung auf einem Monitor möglich. Neben den Koordinaten liegen an der Einheit 3-3 Signale F1 bis Fq an, die beispielsweise Informationen über die Stellung des Beobachters, Parameter des Ausgabesystemes oder den Objektivfocus vorgeben.

Am Ausgang der Einheit 3-3 werden den Signalen eine Vertikal- und Horizontalkomponente (YDi und XDi) eine Luminanzfunktion (LHTi) zugeordnet, um eine Bilddarstellung zu ermöglichen.

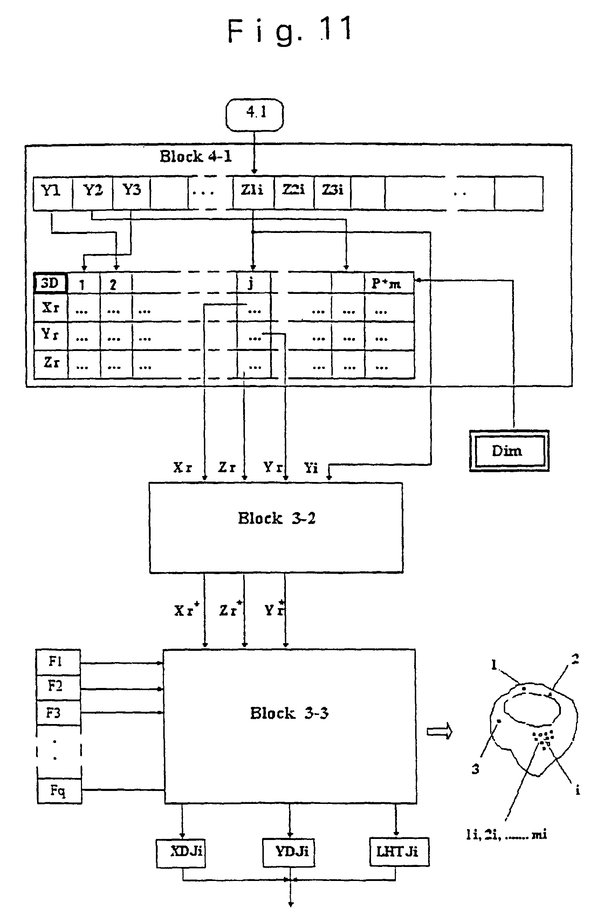

In der Figur 11 ist in einem Blockschaltbild die Ermittlung der Bildwerte in der Umgebung der Stützstellen dargestellt, die prinzipiell dem Verfahren der Zuordnung von Raumkoordinaten in der Einheit 3-1 verläuft. Die für eine geschlossene Oberflächendarstellung benötigten Zwischenwerte (Z1i, Z2i...Z), werden analog zu den oben beschriebenen Verfahren ermittelt, wobei die 3D-Tabelle auf der Grundlage der p Spalten in Block 3-1 in Figur 10 um den Vergrößerungsfaktor m vergrößert wird, also p*m Spalten vorliegen. Die Interpolation zwischen den jeweiligen Punkten erfolgt nach bekannten Algorithmen, im einfachsten Fall durch lineare Interpolation. Schematisch ist die diese Interpolation in der Figur 11 unten rechts veranschaulicht, wo die Bildpunkte li bis mi um den Punkt i herum dargestellt sind. Entsprechend werden die übrigen interpolierten Punkte um die anderen Stützstellen ermittelt.

Die jeweiligen Funktionen zur Berechnung der Werte, also welche Farbwerte dem jeweiligen Signalwert zugeordnet werden, wie die Raumkoordinaten zu dem jeweiligen Wert errechnet werden oder welche Helligkeitswerte vergeben werden, sind in dem Programmspeicher 7 abgelegt und werden auf der Basis empirischer Untersuchungen ermittelt. Die Errechnung der Raumkoordinaten und die Zuordnung der Helligkeitswerte erfolgt parallel.

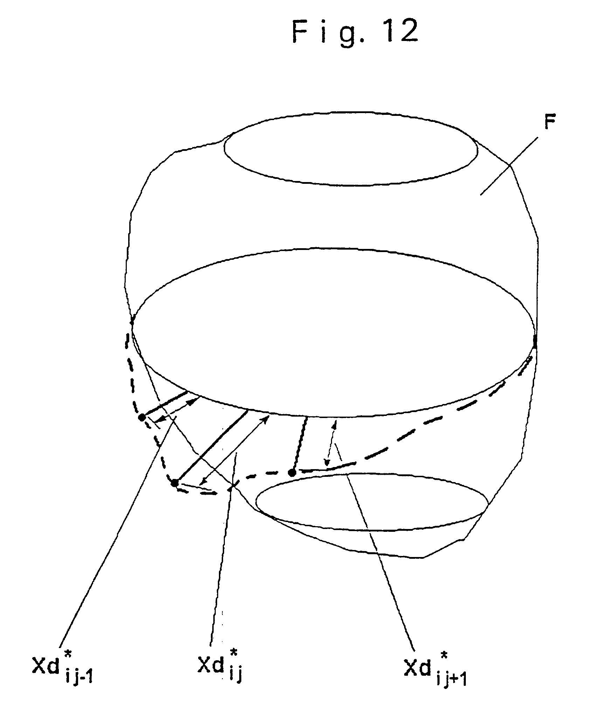

Figur 12 zeigt eine schematische Darstellung der Raumkoordinatenzuordnung in einer ausgewählten Ebene. Ausgehend von einem Grundmodell F werden aufgrund der errechneten Werte $Xd^*_{ij-1}$, $Xd^*_{i1}$ und $Xd^*_{ij+1}$ neue Raumkoordinaten zugeordnet, die als Stützstellen zur Bildung eines geschlossenen Kurven-Kurvenzuges in einer Ebene dienen. Entsprechend werden die Werte für alle Ebenen gebildet, so daß sich eine räumliche Darstellung ergibt.

Auf die oben beschriebene Weise lassen sich die Informationen, die in einem EKG enthalten sind, plastisch und farbig darstellen, wobei die Farbigkeit und Plastizität in Abhängigkeit von medizinischen Fakten erfolgt und keine willkürliche Zuordnung ist. Eine solche Darstellung ist einem Mikroskop vergleichbar, das vorhandene Informationen, die zunächst der unmittelbaren Wahrnehmung entzogen sind, einem Beobachter zugänglich macht. Die Kleinschwankungen innerhalb eines untersuchten EKG-Signals eines "kranken" Herzens unterscheiden sich von den Kleinschwankungen eines gesunden Herzens. Auf der Basis medizinischer Daten werden diese Kleinschwankungen, die bei anderen Untersuchungsverfahren in dem Signalrauschen untergingen, in dem oben beschriebenen Verfahren sichtbar gemacht. Da die vorhandenen aber schwer zu deutenden Informationen eines EKG in einer Art topologischer Darstellung aufbereitet werden, die kleinste Abweichungen sichtbar werden lassen, ist der Begriff Informationsmikroskop angebracht.

Die wie beschrieben ermittelten Bilddaten werden in einer Steuerungseinheit 8 für die jeweilige Auswerte- oder Anzeigeeinheit, beispielsweise einen Farbdrucker oder einen Monitor, aufbereitet und so dem Patienten oder Arzt sichtbar gemacht. Selbstverständlich können solche Bilder gespeichert oder über Datenleitungen übermittelt werden.

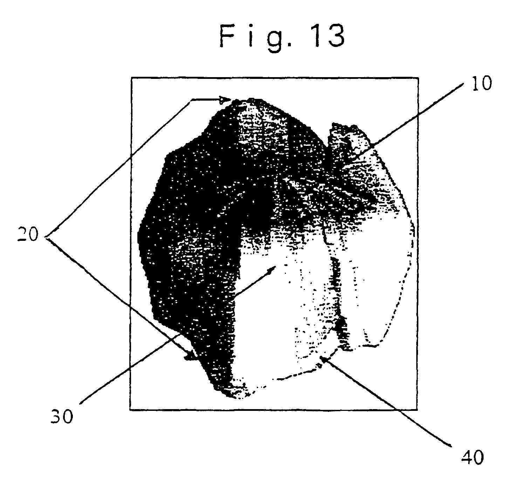

Figur 13 zeigt eine beispielhafte Darstellung eines bearbeiteten EKG in seiner topologischen Form. Aus der Form und Farbe der jeweiligen Bereiche sind verschiedene Aussagen ableitbar. Der Bereich 10 beschreibt das Streßniveau zum Zeitpunkt der Messung. Je nach Belastung, beispielsweise durch körperliche Betätigung, Medikamente oder Sucht- bzw. Genußmittel bilden sich spezifische Veränderungen aus, die insbesondere durch eine Verfärbung sichtbar werden.

Die Bereiche 20 erlauben Rückschlüsse auf die allgemeine Myokardfunktion, während sich bei schweren Erkrankungen die Farbe und die Konturen des Bereiches 30 übergangslos ändern. Im Bereich 40 sind die Funktionen der Herzkammern angesiedelt, so daß hier eventuelle Abnormitäten oder Besonderheiten angezeigt werden.

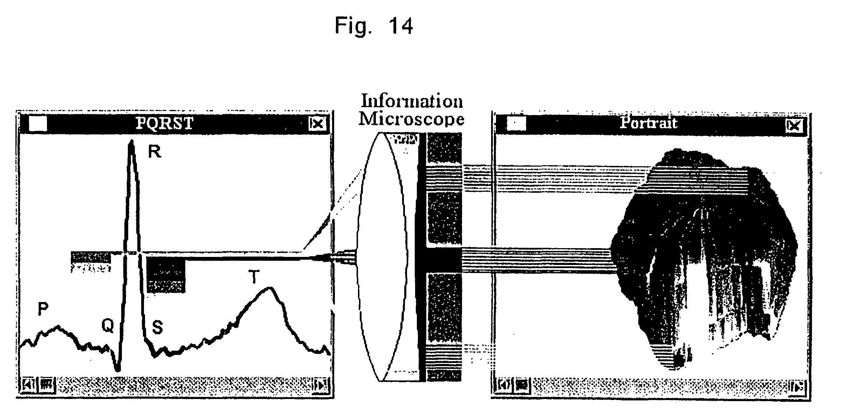

Figur 14 zeigt eine Prinzipdarstellung der Verarbeitung eines EKG-Signals durch die erfindungsgemäße Vorrichtung nach dem obenbeschriebenen Verfahren. Das gemessene EKG-Signal, in der Figur 14 links in der herkömmlichen Kurvendarstellung abgebildet, wird allgemein in die Bereiche PQRST unterteilt. In dem Verfahren werden die stabilen Kleinschwankungen des gemessenen Signals in der als "Informationsmikroskop" bezeichneten Vorrichtung ausgewertet und aufbereitet und so auch dem ungeschulten Auge sichtbar gemacht. Den jeweiligen Bereichen der Kurvendarstellung des EKG-Signals sind Areale auf dem dreidimensionalen topologischen Modell zugeordnet, das in der Figur 14 rechts unter der Überschrift "Portrait" abgebildet ist.

Der Bereich um Q, also die Vorhofkontraktion, findet sich auf dem Portrait in dem Areal oben rechts wieder, der Bereich um S ist am linken Rand angeordnet und die ST-Strekke findet ihre Entsprechung im unteren Areal des Portraits.

Die Vorrichtung, das "Informationsmikroskop" ist als eine Art Vergrößerungsglas dargestellt, um deren Funktion bildhaft darzustellen. Die Kleinschwankungen werden erfaßt, verarbeitet und "vergrößert" und ein topologisches Modell wird gebildet, das sog. Portrait, an dem sich im Gegensatz zu dem herkömmlichen EKG die Veränderungen und der Zustand des Herzens auf einfache Weise ablesen läßt.

Durch das einfache Meßverfahren nach der standardisierten 3-Punkt-Ableitung und die einfache Auswertbarkeit ist mit diesem Verfahren der Darstellung und mit der erfindungsgemäßen Vorrichtung die Möglichkeit gegeben, im privaten Bereich eine Kontrolluntersuchung durchzuführen, die auch einem Laien zumindest Anhaltswerte für weitere Untersuchungen oder Behandlungen gibt.

Im klinischen Bereich können aufgrund der hohen Auflösung und der kurzen und prägnanten Auswertung die Dauer von Erholungsphasen nach Belastungen oder die Auswirkungen von Medikamenten unmittelbar beobachtet werden. Mit 6-Punkt-Ableitungen erhöht sich die Genauigkeit der Messung und die Informationsmenge, so daß qualitative und quantitative Aussagen über den Zustand des Herzens getroffen werden können.

**Patentansprüche**

1. Verfahren zur Darstellung und Überwachung zyklischer Funktionsparameter eines physiologischen Systems, insbesondere elektrokardiographischer Daten, die aus elektronischen Meßsignalen abgeleitet werden, wobei die Daten zu einem Datengrundmodell zusammengefaßt und in ein graphisches Portrait umgewandelt werden, das nach Art eines dreidimensionalen topologischen Modells aufgebaut ist, wobei

    a) ein Analysezyklus durch Ermittlung des zeitlichen Abstandes zumindest zweier sich wiederholender signifikanter Größen festgelegt wird,

    b) die innerhalb dieses Analysezyklus nichtinvasiv aufgenommenen Daten digitalisiert werden,

    c) die digitalisierten Daten einer Speichereinheit (4) zugeführt werden,

    d) den jeweiligen Daten ein Farbcode als eine Funktion der digitalisierten Daten zugeordnet wird, wobei die Zuordnung der Farbcodes und Helligkeitswerte nach dem Grad der Abweichung der aufgenommenen Daten von empirisch ermittelten Vergleichsdaten erfolgt,

e) aus den Daten Raumkoordinaten als Flächenstützstellen ermittelt werden, zwischen denen Bildpunkte zur Erzeugung einer im wesentlichen geschlossenen Fläche interpoliert werden und

f) die errechneten Bildinformationen einer Auswerteoder Ausgabeeinrichtung (9) zugeführt werden.

**dadurch gekennzeichnet,**
**daß** in Schritt d)

- eine Auswahl der auszuwertenden Analysezyklen nach einer Berechnung der Autokorrelationsfunktion der auszuwertenden Analysezyklen und eines Vergleiches mit empirisch ermittelten Vergleichsdaten erfolgt,

- die aufgenommenen Daten eines Analysezyklusses durch einen Hochpaßfilter geleitet, digitalisiert und von den ungefilterten Daten subtrahiert werden, die so gewonnenen Daten entsprechend ihrem Wert gruppiert werden und die Reihenfolge der Umgruppierung für jedes physiologische System gespeichert wird,

- die Reihenfolge der Umgruppierung im Verlauf einer Referenzmessung ermittelt und auf nachfolgende Analysezyklen zur Ermittlung von Veränderungen des physiologischen Systems angewendet wird.

2. Verfahren nach Anspruch 1, **dadurch gekennzeichnet, daß** Aktionspotentiale bei einer Herzuntersuchung nach einer standardisierten 3-Punkt-Ableitung ermittelt werden.

3. Verfahren nach Anspruch 2, **dadurch gekennzeichnet, daß** ausgehend vom Abstand zweier R-Impulse eines Elektrokardiogrammes ein Analysezyklus festgelegt wird, der die signifikanten Aktionspotentiale des Herzmuskels erfaßt.

4. Verfahren nach mindestens einem der vorangehenden Ansprüche, **dadurch gekennzeichnet, daß** die Daten über ein Zeitintervall ermittelt werden, das ein Vielfaches eines Analysezyklus umfaßt.

5. Verfahren nach Anspruch 1, **dadurch gekennzeichnet, daß** die Vergleichsdaten aus einem elektronischen Speicher (7) abgerufen werden.

6. Verfahren zumindest einem der vorangehenden Ansprüche, **dadurch gekennzeichnet, daß** alle vollständigen Analysezyklen eines Zeitintervalles bei der Bildberechnung berücksichtigt werden.

7. Verfahren nach mindestens einem der vorangehenden Ansprüche, **dadurch gekennzeichnet, daß** Störgrößen durch eine Korrelation der aufgenommenen Daten mehrerer Analysezyklen eliminiert werden.

8. Verfahren nach zumindest einem der voranstehenden Ansprüche, **dadurch gekennzeichnet, daß** die aufgenommenen Daten über Datenleitungen an Diagnosezentren übermittelt werden.

9. Verfahren nach zumindest einem der voranstehenden Ansprüche, **dadurch gekennzeichnet, daß** die Bilddarstellung mit einer Therapieempfehlung hinterlegt wird.

10. Vorrichtung zur Darstellung und Überwachung zyklischer Funktionsparameter eines physiologischen Systems, insbesondere elektrokardiographischer Daten, wobei die Daten zu einem Datengrundmodell zusammengefaßt und in ein graphisches Portrait umgewandelt werden, das nach Art eines dreidimensionalen topologischen Modells aufgebaut ist,
mit Mitteln (1) zur Datenaufnahme und Umwandlung in elektrische Signale,
einer Vorrichtung (2) zur Festlegung eines Analysezyklus durch Ermittlung des zeitlichen Abstandes zumindest zweier sich wiederholender signifikanter Größen,
einem Analog-Digitalwandler (3) zur Digitalisierung innerhalb dieses Analysezyklus nichtinvasiv aufgenommener Daten
sowie einer Speichereinheit (4) zur Speicherung der digitalisierten Daten
einer Auswerteeinheit (5, 6, 7) mit einer Auswertesoftware zur Zuordnung eines Farbcodes als eine Funktion der digitalisierten Daten zugeordnet wird, wobei die Zuordnung der Farbcodes und Helligkeitswerte nach dem Grad der Abweichung der aufgenommenen Daten von empirisch ermittelten Vergleichsdaten erfolgt,
Mitteln zur Ermittlung von Raumkoordinaten als Flächenstützstellen, zwischen denen Bildpunkte zur Erzeugung einer im wesentlichen geschlossenen Fläche interpoliert werden und Erzeugung eines dreidimensionalen, farbigen Bildes mit einer geschlossenen Oberfläche auf der Grundlage der aufgenommenen und ausgewerteten Signale, einer Steuerungseinheit (8) für die Aufbereitung der ermittelten Bildpunkte sowie einer Auswerteoder Ausgabeeinrichtung (9).
**dadurch gekennzeichnet,**

**daß** die Auswerteeinheit (5, 6, 7)

- die aufgenommenen Daten eines Analysezyklusses durch einen Hochpaßfilter leitet, digitalisiert und von den ungefilterten Daten subtrahiert, die so gewonnenen Daten entsprechend ihrem Wert gruppiert und die Reihenfolge der Umgruppierung für jedes physiologische System der Speichereinheit (4) zuführt,

- die Reihenfolge der Umgruppierung im Verlauf einer Referenzmessung ermittelt und auf nachfolgende Analysezyklen zur Ermittlung von Veränderungen des physiologischen Systems anwendet,

- eine Auswahl der auszuwertenden Analysezyklen nach einer Berechnung der Autokorrelationsfunktion der auszuwertenden Analysezyklen und eines Vergleiches mit empirisch ermittelten Vergleichsdaten durchführt.

11. Vorrichtung nach Anspruch 10, **dadurch gekennzeichnet, daß** an der Auswerteeinheit (5) eine Schnittstelle zur externen Datenübermittlung vorgesehen ist.

12. Vorrichtung nach Anspruch 10 oder 11, **dadurch gekennzeichnet, daß** ein Verstärker der elektrischen Signale vorgesehen ist.

13. Vorrichtung nach einem der Ansprüche 10 bis 12, **dadurch gekennzeichnet, daß** die Vorrichtungen zur Datenaufnahme als Elektroden, Drucksensoren, Durchflußmengenmesser oder optische Sensoren ausgebildet sind.

**Claims**

1. Method for representing and monitoring cyclical function parameters of a physiological system, in particular electrocardiographic data, which are derived from electronic measuring signals, the data being combined to form a basic data model and being converted into a graphical portrait which is constructed in the manner of a three-dimensional topological model, in which case

    a) an analysis cycle is fixed by determining the temporal spacing of at least two repeating significant variables,

    b) the data non-invasively acquired during this analysis cycle are digitized,

    c) the digitized data are fed to a storage unit (4),

    d) the respective data are assigned a colour code as a function of the digitized data, the assignment of the colour codes and brightness values being performed according to the degree of deviation of the acquired data from empirically determined reference data,

    e) space coordinates are determined from the data as surface interpolation points between which pixels for generating an essentially closed surface are interpolated, and

    f) the image information calculated is fed to an evaluation or output device (9),

    **characterized in that**
    in step d)

    - a selection of the analysis cycles to be evaluated is performed after calculating the autocorrelation function of the analysis cycles to be evaluated and after a comparison with empirically determined reference data,

    - the acquired data of an analysis cycle are led through a high-pass filter, digitized and subtracted from the unfiltered data, the data thus obtained are grouped according to their value and the sequence of the regrouping is stored for each physiological system,

    - the sequence of the regrouping is determined in the course of a reference measurement and applied to subsequent analysis cycles in order to determine variations in the physiological system.

2. Method according to Claim 1, **characterized in that** action potentials in the case of a heart examination are determined using a standardized 3-point recording.

3. Method according to Claim 2, **characterized in that** starting from the interval of two R pulses of an electrocardiogram, an analysis cycle is fixed which detects the significant action potentials of the heart muscle.

4. Method according to at least one of the preceding claims, **characterized in that** the data are determined over a time interval which covers a multiple of an analysis cycle.

5. Method according to Claim 1, **characterized in that** the reference data are called from an electronic memory (7).

6. Method according to at least one of the preceding claims, **characterized in that** all complete analysis cycles of a time interval are taken into account in the image calculation.

7. Method according to at least one of the preceding claims, **characterized in that** noise quantities are eliminated by correlation of the acquired data of a plurality of analysis cycles.

8. Method according to at least one of the preceding claims, **characterized in that** the acquired data are transmitted to diagnostic centres via data lines.

9. Method according to at least one of the preceding claims, **characterized in that** the image representation is stored with a therapy recommendation.

10. Device for representing and monitoring cyclical function parameters of a physiological system, in particular electrocardiographic data, the data being combined to form a basic data model and being converted into a graphical portrait which is constructed in the manner of a three-dimensional topological model,
having means (1) for data acquisition and conversion into electric signals,
having a device (2) for fixing an analysis cycle by determining the temporal spacing of at least two repeating significant variables,
having an analogue-to-digital converter (3) for digitizing data acquired non-invasively within this analysis cycle
and having a storage unit (4) for storing the digitized data
having an evaluation unit (5, 6, 7) with evaluation software for assigning a colour code as a function of the digitized data, the assignment of the colour codes and brightness values being performed according to the degree of deviation of the recorded data from empirically determined reference data,
having means for determining space coordinates as surface interpolation points between which pixels for generating an essentially closed surface are interpolated, and for generating a three-dimensional, coloured image with a closed surface on the basis of the acquired and evaluated signals, a control unit (8) for conditioning the pixels determined, and an evaluation or output unit (9),
**characterized in that**
the evaluation unit (5, 6, 7)

- leads the acquired data of an analysis cycle through a high-pass filter, digitizes them and subtracts them from the unfiltered data, groups the data thus obtained according to their value, and feeds the sequence of the regrouping for each physiological system to the storage unit (4),

- determines the sequence of the regrouping in the course of a reference measurement and applies it to subsequent analysis cycles in order to determine variations in the physiological system,

- carries out a selection of the analysis cycles to be evaluated after calculating the autocorrelation function of the analysis cycles to be evaluated and after a comparison with empirically determined reference data.

11. Device according to Claim 10, **characterized in that** an interface for external data transmission is provided at the evaluation unit (5).

12. Device according to Claim 10 or 11, **characterized in that** an amplifier of the electric signals is provided.

13. Device according to one of Claims 10 to 12, **characterized in that** the devices for data acquisition are designed as electrodes, pressure sensors, flowrate meters or optical sensors.

## Revendications

1. Procédé de visualisation et de surveillance de paramètres fonctionnels cycliques d'un système physiologique, notamment de données électrocardiographiques déduites de signaux de mesure électroniques, les données étant regroupées dans un modèle de base de données et transformées en un portrait graphique construit à la manière d'un modèle topologique tridimensionnel, où

    a) un cycle d'analyse est défini par la détermination de l'écart dans le temps d'au moins deux grandeurs significatives répétitives,
    b) les données captées de manière non invasive au cours de ce cycle d'analyse sont numérisées,
    c) les données numérisées sont transmises à une unité de mémoire (4),
    d) aux données respectives est affecté un code couleur comme une fonction des données numérisées, l'affectation des codes couleur et des valeurs de luminosité étant réalisée selon le degré de l'écart des données captées par rapport à des données de comparaison empiriques,
    e) à partir des données des coordonnées spatiales sont déterminées comme points d'appui superficiels entre lesquels des points d'image sont interpolés pour générer une surface pour l'essentiel fermée, et
    f) les informations d'image calculées sont transmises à un dispositif d'exploitation ou d'émission (9),

**caractérisé en ce que** dans l'étape d)

- une sélection des cycles d'analyse est effectuée après un calcul de la fonction d'autocorrélation des cycles d'analyse à exploiter et après une comparaison avec des données de comparaison empiriques,
- les données captées d'un cycle d'analyse sont passées dans un filtre passe-haut, numérisées et soustraites des données non filtrées, les données ainsi obtenues sont regroupées en fonction de leur valeur, et l'ordre du regroupement est mémorisé pour chaque système physiologique,
- l'ordre du regroupement est déterminé au cours d'une mesure de référence et appliqué sur des cycles d'analyse ultérieurs pour déterminer des modifications du système physiologique.

2. Procédé selon la revendication 1, **caractérisé en ce que** des potentiels d'action sont déterminés à l'occasion d'un examen cardiaque selon une dérivation à trois points standardisée.

3. Procédé selon la revendication 2, **caractérisé en ce qu'**à partir d'un écart de deux impulsions R d'un électrocardiogramme un cycle d'analyse est défini, qui détecte les potentiels d'action significatifs du myocarde.

4. Procédé selon au moins l'une des revendications précédentes, **caractérisé en ce que** les données sont déterminées sur un intervalle de temps comprenant un multiple d'un cycle d'analyse.

5. Procédé selon la revendication 1, **caractérisé en ce que** les données de comparaison sont appelées d'une mémoire électronique (7).

6. Procédé selon au moins l'une des revendications précédentes, **caractérisé en ce que** tous les cycles d'analyse complets d'un intervalle de temps sont pris en compte pour le calcul de l'image.

7. Procédé selon au moins l'une des revendications précédentes, **caractérisé en ce que** des grandeurs parasites sont éliminées par une corrélation des données captées de plusieurs cycles d'analyse.

8. Procédé selon au moins l'une des revendications précédentes, **caractérisé en ce que** les données captées sont transmises à des centres de diagnostic par l'intermédiaire de lignes de données.

9. Procédé selon au moins l'une des revendications précédentes, **caractérisé en ce que** la visualisation d'image est mémorisée avec des consignes de thérapie.

10. Dispositif de visualisation et de surveillance de paramètres fonctionnels cycliques d'un système physiologique, notamment de données électrocardiographiques, les données étant regroupées en un modèle de base de données et transformées en un portrait graphique construit à la manière d'un modèle topologique tridimensionnel, comprenant des moyens (1) pour capter et pour transformer de données en signaux électriques, un dispositif (2) pour définir un cycle d'analyse par la détermination de l'écart dans le temps d'au moins deux grandeurs significatives répétitives, un convertisseur analogique-numérique (3) pour la numérisation de données d'une façon non invasive captées au cours de ce cycle d'analyse, ainsi qu'une unité de mémoire (4) pour mémoriser les données numérisées, une unité d'exploitation (5, 6, 7) avec un logiciel d'exploitation pour affecter un code couleur en tant qu'une fonction des données numérisées, l'affectation des codes couleur et des valeurs de luminosité étant réalisée selon le degré de l'écart des données captées par rapport à des données de comparaison empiriques, des moyens pour déterminer des coordonnées spatiales comme points d'appui superficiels entre lesquels des points d'image sont interpolés pour générer une surface pour l'essentiel fermée, et génération d'une image couleur tridimensionnelle avec une surface fermée à partir des signaux captés et exploités, une unité de commande (8) pour le traitement des points d'images déterminés, ainsi qu'une unité d'exploitation ou d'émission (9), **caractérisé en ce que** l'unité d'exploitation (5, 6, 7)

- passe les données captées d'un cycle d'analyse dans un filtre passe-haut, les numérise et les soustrait des données non filtrées, regroupe les données ainsi obtenues en fonction de leur valeur et transmet l'ordre du regroupement pour chaque système physiologique à l'unité de mémoire (4),
- détermine l'ordre du regroupement au cours d'une mesure de référence et l'applique sur des cycles d'analyse ultérieurs pour déterminer des modifications du système physiologique,
- fait une sélection des cycles d'analyse à exploiter selon un calcul de la fonction d'autocorrélation des cycles d'analyse à exploiter et une comparaison avec des données de comparaison empiriques.

11. Dispositif selon la revendication 10, **caractérisé en ce qu'**une interface pour la transmission de données externe est prévue sur l'unité d'exploitation (5).

**12.** Dispositif selon la revendication 10 ou 11, **caractérisé en ce qu'**un amplificateur des signaux électriques est prévu.

**13.** Dispositif selon l'une des revendications 10 à 12, **caractérisé en ce que** les dispositifs pour capter les données sont configurées en forme d'électrodes, de capteurs de pression, de débitmètres ou de capteurs optiques.

# Fig. 1

# Fig. 2

# Fig. 3

| Meßwert-aufnehmer | → | Bearbeitungs-einrichtung | → | A / D |
|---|---|---|---|---|

1    2    3

| Programm-speicher | ← | Rechen-einheit | ← | Speicher-einheit |
|---|---|---|---|---|

7    6    4    5

| Steuerungs-einheit | → | Ausgabe-einrichtung |
|---|---|---|

8    9

Fig. 4

# F i g. 4a

# F i g. 4b

Fig. 5

Fig. 6

# F i g. 7

```
                          ┌──────┐
                          │  2.1 │
                          └──────┘
                              │
                              ▼
┌──────┬──────┬──────┬──────┬ ─ ─ ─ ─ ─ ─ ─ ─ ┬──────────┐
│  Yi  │ Xdij*│ Xdij*│ Xdij*│                 │  Xdij *  │
└──────┴──────┴──────┴──────┴ ─ ─ ─ ─ ─ ─ ─ ─ ┴──────────┘
                              │
                              ▼
                    ┌──────────────────┐
                    │    Block 2-1     │
                    └──────────────────┘
                              │
                              ▼
┌──────────┬──────────┬──────────┬ ─ ─ ─ ─ ─ ─ ─ ┬──────────┐
│ Kor_i[]  │  K_i(1)  │  K_i(2)  │               │  K_i(p)  │
└──────────┴──────────┴──────────┴ ─ ─ ─ ─ ─ ─ ─ ┴──────────┘

┌──────────┬──────────┬──────────┬ ─ ─ ─ ─ ─ ─ ─ ┬──────────┐
│ Kor_E[]  │  K_E(1)  │  K_E(2)  │               │  K_E(p)  │
└──────────┴──────────┴──────────┴ ─ ─ ─ ─ ─ ─ ─ ┴──────────┘
                              │
                              ▼
                    ┌──────────────────┐
                    │    Block 2-2     │
                    └──────────────────┘
                              │
                              ▼
                          ◇ Kor_i[]=
                            Kor_E[] ?
                         Ja         Nein
```

Kor_i[]=
Kor_E[] ?

Ja      Nein

Dim

Block 2-3

Yi*  ⊗

Yi

# Fig. 8

2.2

Yi

Block 2-4

| # | Col(#) |
|---|--------|
| 1 | Col(1) |
| 2 | Col(2) |
| 3 | Col(3) |
|   |        |
| i | Col(i) |
|   |        |
|   |        |
| V | Col(K) |

| 1 | 1 | 2 | | | | | 1024 |
|---|---|---|---|---|---|---|------|
| R | ... | ... | ... | | ... | ... | ... |
| G | ... | ... | ... | | ... | ... | ... |
| B | ... | ... | ... | | ... | ... | ... |

| i | 1 | 2 | | | | | 1024 |
|---|---|---|---|---|---|---|------|
| R | ... | ... | ... | | ... | ... | ... |
| G | ... | ... | ... | | ... | ... | ... |
| B | ... | ... | ... | | ... | ... | ... |

| V | 1 | 2 | | | | | 1024 |
|---|---|---|---|---|---|---|------|
| R | ... | ... | ... | | ... | ... | ... |
| G | ... | ... | ... | | ... | ... | ... |
| B | ... | ... | ... | | ... | ... | ... |

Bi        Gi        Ri

21

F i g. 9

EP 1 047 987 B1

# F i g. 10

# F i g. 11

# Fig. 12

F

$Xd^*_{ij-1}$    $Xd^*_{ij}$    $Xd^*_{ij+1}$

EP 1 047 987 B1

# F i g. 13

26

Fig. 14

EP 1 047 987 B1